# EUROPÄISCHE PATENTSCHRIFT

(11) **EP 3 137 020 B1**
(45) Veröffentlichungstag und Bekanntmachung des Hinweises auf die Patenterteilung: **31.01.2018**
(21) Anmeldenummer: 15729756.5
(22) Anmeldetag: 29.04.2015
(51) Int. Cl.: A61F 2/66, A61F 2/68, A61F 2/74, A61F 2/76

(54) **PROTHESE**
PROSTHESIS
PROTHÈSE

(30) Priorität: 30.04.2014 DE 102014006228
(43) Veröffentlichungstag der Anmeldung: 08.03.2017
(73) Patentinhaber: Otto Bock Healthcare GmbH, 37115 Duderstadt (DE)
(72) Erfinder: PUSCH, Martin, 37115 Duderstadt (DE); KALTENBORN, Sven, 37115 Duderstadt (DE); MOSLER, Lüder, 37115 Duderstadt (DE); GEHRMANN, Georg, 37085 Göttingen (DE); MÖNICKE, Carsten, 37115 Duderstadt (DE)
(74) Vertreter: Plöger, Jan Manfred
(86) Internationale Anmeldenummer: PCT/EP2015/059365
(87) Internationale Veröffentlichungsnummer: WO 2015/165981

(56) Entgegenhaltungen:
- WO-A1-97/12567
- WO-A1-2008/071975
- WO-A1-2014/039885

## Beschreibung

Die Erfindung betrifft eine Prothese mit (a) einem Unterschenkel-Anschlussteil, (b) einem Fußteil, (c) einem Verbindungselement mit Gelenkfunktion, das das Unterschenkel-Anschlussteil gemäß einem Knöchelgelenk mit dem Fußteil verbindet, und (d) einer Freigabevorrichtung, mittels der das Fußteil in eine Arretierstellung, in der das Fußteil und das Unterschenkel-Anschlussteil zumindest bezüglich einer Dorsalextensionsbewegung des Fußteils relativ zum Unterschenkel-Anschlussteil drehstarr miteinander verbunden sind, und in eine Freigabestellung, in der das Fußteil relativ zum Unterschenkel-Anschlussteil drehbar ist, bringbar ist.

Eine derartige Prothese kann beispielsweise als reiner Prothesenfuß oder als Knie-Fuß-Prothese ausgebildet sein und dient als Ersatz zumindest für den natürlichen Fuß. Besonders bei Patienten mit schwächeren muskulären und koordinativen Fähigkeiten ist es wichtig zu verhindern, dass der Patient stolpert. Es sind Prothesen bekannt, die über einen Mikroprozessor verfügen, der mit Beschleunigungssensoren verbunden ist und aus den Signalen der Beschleunigungssensoren ermittelt, in welcher Phase im Gangzyklus der Patient sich gerade befindet. Derartige Prothesen sind aufwändig zu fertigen und zu warten, so dass nicht alle Patienten Zugang zu solchen Prothesen haben.

Eine Prothese ist aus der US 8,709,097 B2 bekannt, bei der ein Mikroprozessor anhand von Sensordaten erfasst, wann der Gangablauf so weit fortgeschritten ist, dass das Gelenk freigeschaltet werden muss. Nachteilig an einer derartigen Prothese ist der hohe Aufwand bei der Fertigung und bei der Wartung. Aus der WO 2014/ 039 885 A1 ist eine Prothese bekannt, bei der ebenfalls mittels eines Mikroprozessors und einer Vielzahl von Sensoren der Ablauf erfasst wird. In der Steuerung können Schwellenwerte hinterlegt werden, anhand der die Aktoren der Prothese angesteuert werden. Auch ein derartiges System ist aufwendig zu fertigen und zu warten.

Die US 2013/0006386 A1 beschreibt eine Fußprothese, die eine Kupplung aufweist. Diese Kupplung blockiert unter Last und löst sich bei Entlastung, um eine Anpassung an Steigungen und Gefälle zu ermöglichen.

Die WO 2008/071975 A1 betrifft eine Fußprothese mit einer Knöchelbaugruppe und einer Unterschenkelkomponete. Die Knöchelbaugruppe ist mit einem Gelenkmechanismus ausgestattet, der dazu eingerichtet ist, eine begrenzte und gedämpfte Schwenkbewegung einer Unterschenkelkomponente relativ zu der Fußprothese zu ermöglichen.

Der Erfindung liegt die Aufgabe zugrunde, eine Prothese vorzuschlagen, die zu einer geringen Wahrscheinlichkeit des Stolperns führt und einfach realisierbar ist.

Die Erfindung löst das Problem durch eine oben genannte Prothese, bei der die Freigabevorrichtung ausgebildet ist zum, insbesondere passiven, Umschalten von der Arretierstellung in die Freigabestellung, wenn ein Knöchel- Drehmoment im Verbindungselement oberhalb eines einstellbaren Drehmoment-Schwellenwerts liegt, der auch als Dorsalextensions-Schwellenwert bezeichnet werden könnte.

Vorteilhaft an einer derartigen Prothese ist, dass der Patient sicher stehen kann. Im Stand liegt ein geringes Knöchel-Drehmoment an, das unterhalb des Drehmoment-Schwellenwerts liegt. Aus diesem Grund ist die Freigabevorrichtung in der Arretierstellung und der Patient steht sicher auf der Prothese.

Beginnt der Patient zu laufen, überschreitet das Knöchel-Drehmoment den voreingestellten Drehmoment-Schwellenwert und die Freigabevorrichtung kommt in ihre Freigabestellung. Das Fußteil führt relativ zum Unterschenkel-Anschlussteil eine Dorsalextensionsbewegung aus. In anderen Worten bewegt sich die Fußspitze auf das Knie des Patienten zu. In der nachfolgenden Schwungphase vergrößert das den Abstand der Fußspitze vom Boden, was die Stolpergefahr reduziert.

Vorteilhaft ist zudem, dass mit der erfindungsgemäßen Prothese ansteigende Rampen einfacher bewältigt werden können. Bei ansteigenden Rampen ist in der Schwungphase der Abstand zwischen der Prothese und dem Boden verringert, was die Stolpergefahr erhöht. Bei der erfindungsgemäßen Prothese ist der Bodenabstand vergrößert. Das verringert die Gefahr des Stolperns, wenn der Patient eine ansteigende Rampe hinaufgeht. Weiterhin erleichtert die Dorsalextensionsstellung beim Ende des Schritts das Heraufgehen von Rampen.

Im Rahmen der vorliegenden Beschreibung wird unter dem Unterschenkel-Anschlussteil insbesondere eine Vorrichtung verstanden, die ausgebildet ist zum direkten oder indirekten mechanischen Verbinden mit einem künstlichen oder natürlichen Unterschenkel. Es ist also möglich, nicht aber notwendig, dass ein künstlicher Unterschenkel und/oder ein künstliches Kniegelenk vorhanden ist, der unlösbar oder lösbar mit dem Unterschenkel-Anschlussteil verbunden ist.

Unter dem Fußteil wird insbesondere derjenige Teil der Prothese verstanden, der die Funktion des menschlichen unteren Fußes übernimmt. Insbesondere kann der Fußteil eine Fußkosmetik umfassen, die der Prothese eine Anmutung eines natürlichen Fußes gibt. Eine derartige Fußkosmetik ist jedoch entbehrlich.

Unter dem Verbindungselement wird insbesondere eine Verbindung zwischen dem Unterschenkel-Anschlussteil und dem Fußteil verstanden, die eine Relativbewegung der beiden Komponenten erlaubt, die der Bewegung eines natürlichen Fußes entspricht. Das Verbindungselement kann ein Drehgelenk aufweisen oder durch ein Drehgelenk gebildet sein, das eine feste Drehachse aufweist. Diese Lage der Drehachse kann vom Drehwinkel des Drehgelenks unabhängig sein.

Es ist aber auch möglich, dass das Verbindungselement mehrere Teil-Gelenke aufweist und eine momentane Drehachse hat, das heißt, dass sich die Drehachse, mittels der die momentane Drehung des Fußteils relativ zum Unterschenkel-Anschlussteil beschreibbar ist, sich mit der Zeit und/oder dem Fortschritt der Bewegung im Gangzyklus ändert. Es ist auch möglich, dass das Verbindungselement zumindest teilweise als Festkörpergelenk aufgebaut ist. Die meisten Prothesen zielen darauf ab, den natürlichen Bewegungsablauf möglichst gut zu imitieren. Da der menschliche Fuß mehrere Teil-Gelenke hat, besitzen viele Prothesen ein Verbindungselement, das eine mit der Zeit in seiner Lage veränderliche Drehachse besitzt. Die Erfindung bezieht sich auch auf Prothesen mit solchen Verbindungselementen.

Unter dem Knöchel-Drehmoment wird insbesondere das Drehmoment verstanden, das sich errechnet aus dem momentanen Hebelarm und der am Fußteil angreifenden Kraft, jeweils in Bezug auf die, gegebenenfalls momentane, Drehachse. Das Knöchel-Drehmoment ist positiv, wenn eine Druckkraft an einer Fußspitze des Fußteils anliegt. In anderen Worten ist das Knöchel-Drehmoment positiv für eine Kraft, die das Fußteil in eine Dorsalextensionsbewegung drückt. Für eine Kraft, die das Fußteil in eine Plantarflexionsbewegung drückt, ist das Knöchel-Drehmoment negativ.

Genau dann, wenn das Fußteil mittels der Freigabevorrichtung in der Arretierstellung ist, ist auch die Freigabevorrichtung in ihrer Arretierstellung. Analog ist die Freigabevorrichtung genau dann in ihrer Freigabestellung, wenn das Fußteil in der Freigabestellung ist.

Es ist möglich und stellt eine bevorzugte Ausführungsform dar, dass die Freigabevorrichtung in der Arretierstellung auch eine Plantarflexionsbewegung des Fußteils unterbindet. Alternativ ist aber auch möglich, dass die Freigabevorrichtung in der Arretierstellung eine Plantarflexionsbewegung ermöglicht, insbesondere dann, wenn das Knöchel-Drehmoment einen voreingestellten Plantarflexions-Schwellenwert, der kleiner als null ist, unterschreitet. In anderen Worten stellt es eine bevorzugte Ausführungsform dar, dass das Fußteil in der Arretierstellung eine Plantarflexionsbewegung ausführen kann, wenn das Knöchel-Drehmoment einen Plantarflexions-Schwellenwert unterschreitet. Der Plantarflexions-Schwellenwert beträgt vorzugsweise höchstens ein Fünftel, insbesondere höchstens ein Zehntel, des Dorsalextensions-Schwellenwerts.

Unter dem Merkmal, dass die Freigabevorrichtung in eine Freigabestellung, in der das Fußteil relativ zum Unterschenkel-Anschlussteil drehbar ist, bringbar ist, wird insbesondere verstanden, dass die Freigabevorrichtung in der Freigabestellung eine Dorsalextensionsbewegung des Fußteils relativ zum Unterschenkel-Anschlussteil erlaubt.

Unter dem Merkmal, dass mittels der Freigabevorrichtung das Fußteil in eine Arretierstellung, in der das Fußteil und das Unterschenkel-Anschlussteil zumindest bezüglich einer Dorsalextensionsbewegung drehstarr miteinander verbunden sind, bringbar ist, wird insbesondere verstanden, dass die Freigabevorrichtung in der Arretierstellung eine Dorsalextensionsbewegung unterbindet, solange nicht das angelegte Knöchel-Drehmoment den Schwellenwert überschreitet.

Unter dem Merkmal, dass die Freigabevorrichtung ausgebildet ist zum Umschalten von der Arretierstellung in die Freigabestellung, wenn eine zu einem Knöchel-Drehmoment im Verbindungselement korrelierende Größe oberhalb eines voreingestellten Schwellenwerts liegt, wird insbesondere verstanden, dass die Freigabevorrichtung so ausgebildet ist, dass ausschließlich ein Überschreiten des Schwellenwerts eine wesentliche Dorsalextensionsbewegung des Fußteils bewirkt. Es ist möglich, dass das Fußteil eine Dorsalextensionsbewegung ausführen kann, wenn die Freigabevorrichtung in der Arretierstellung ist, diese Dorsalextensionsbewegung ist dann aber so stark gehemmt, dass sie vom Patienten nicht oder kaum wahrgenommen wird.

Gemäß einer bevorzugten Ausführungsform ist eine Plantarflexionsbewegung über die Nullstellung hinaus blockiert.

Unter dem Merkmal, dass das Fußteil relativ zum Unterschenkel-Anschlussteil drehbar ist, wird insbesondere verstanden, dass die Freigabevorrichtung eine Bewegung zwischen Fußteil und Unterschenkel-Anschlussteil so wenig behindert, dass das Fußteil im normalen Gangzyklus eine Dorsalextensionsbewegung ausführt oder ausführen kann. Vorzugsweise ist die Freigabevorrichtung so ausgebildet, dass sie einer Bewegung des Fußteils relativ zum Unterschenkel-Anschlussteil in der Arretierstellung einen zumindest dreifach so hohen Widerstand entgegensetzt wie in der Freigabestellung.

Vorzugsweise verbindet das Verbindungselement das Unterschenkel-Anschlussteil wie ein Knöchelgelenk mit dem Fußteil. Hierunter ist insbesondere zu verstehen, dass das Verbindungselement die Bewegung des menschlichen Knöchelgelenks nachbildet. Es kann sich daher bei dem Verbindungselement um ein Drehgelenk handeln, das ist aber nicht notwendig.

Vorzugsweise ist das Verbindungselement so ausgebildet, dass es einer Dorsalextensions- und/oder Plantarflexionsbewegung einen, insbesondere um zumindest den Faktor 10, geringeren Widerstand entgegensetzt als einer Medial- oder Lateralbewegung.

Unter dem Merkmal, dass die Freigabevorrichtung ausgebildet ist zum passiven Umschalten von der Arretierstellung in die Freigabestellung, wird insbesondere verstanden, dass das Umschalten ohne einen Aktor erfolgt. Ein Aktor ist ein Bauteil, das von außen steuerbar durch Energieeinsatz ein anderes Bauteil bewegt. In anderen Worten handelt es sich beim passiven Umschalten um ein Wechseln von der Arretierstellung in die Freigabestellung, die ohne Zufuhr gespeicherter und/oder elektrischer Energie erfolgt. Es ist möglich, nicht aber notwendig, dass beim Umschalten ein Schaltzustand eines Bauteils, beispielsweise eines Ventils, geändert wird. Es ist auch möglich, dass das Umschalten dadurch erfolgt, dass ein Bauteil erst beim Überschreiten des voreingestellten Drehmoment-Schwellenwerts eine Bewegung ausführt und sich unterhalb dieses Drehmoment-Schwellenwertes starr verhält. Beispielsweise kann die Freigabevorrichtung eine gegen einen Anschlag vorgespannte Feder aufweisen.

Es ist möglich, aber entbehrlich, dass der Schwellenwert für das Knöchel- Drehmoment als Drehmoment bekannt ist. Insbesondere ist es möglich, dass dieser Drehmoment-Schwellenwert hin zu größeren und/oder kleineren Drehmoment-Schwellenwerten veränderbar ist, ohne dass der absolute Wert des Drehmoments bekannt ist. Wie groß der jeweilige Drehmoment-Schwellenwert in Newtonmeter ist, kann also bekannt sein, das ist aber nicht notwendig.

Der Drehmoment-Schwellenwert kann insbesondere ermittelt werden, indem eine lotrecht wirkende Kraft auf das Unterschenkel-Anschlussteil ausgeübt, danach das Unterschenkel-Anschlussteil gemäß dem Neigungsverlauf im Gangzyklus geneigt und der Punkt erfasst wird, in dem die Prothese die Kraft in den Boden einleitet. Das (äußere) Vektorprodukt aus der Kraft und dem Hebelarm zu dem Zeitpunkt, in dem die Freigabevorrichtung aus der Arretierstellung in die Freigabestellung schaltet, entspricht dem Schwellenwert.

Die Freigabevorrichtung kann parallel oder seriell zu einem weiteren federnden Element der Prothese angeordnet sein. Beispielsweise ist das federnde Element Teil des Fußteils. Das federnde Element umfasst vorzugsweise eine Karbonfeder. Unter einer parallelen Anordnung wird verstanden, dass sich die mechanischen Widerstände der Freigabevorrichtung und des federnden Elements addieren. Unter einer seriellen Anordnung wird verstanden, dass sich die Federwege der Freigabevorrichtung und des federnden Elements addieren.

Die Freigabevorrichtung kann druckbelastet oder zugbelastet angeordnet sein. Ist die Freigabevorrichtung druckbelastet, so wird sie am Ende der Standphase zusammengedrückt. Ist die Freigabevorrichtung zugbelastet, so wird sie am Ende der Standphase auseinandergezogen.

Gemäß einer bevorzugten Ausführungsform hat die Prothese eine mechanische Vorspannvorrichtung, mittels der das Fußteil in die Dorsalextensions-Stellung vorgespannt ist, beispielsweise eine Vorspannfeder, die so angeordnet sein kann, dass sie beim Fersenauftritt im Anschlag ist.

Umfasst die Prothese keine Vorspannfeder, was ebenfalls eine vorteilhafte Ausführungsform ist, so wird die Freigabevorrichtung durch die Plantarflexion beim Fersenauftritt in ihre Neutralposition gebracht. Bei der nachfolgenden Dorsalextension unterstützt die Vorspannfeder die Dorsalextensionsbewegung des Fußteils.

Gemäß einer bevorzugten Ausführungsform ist die Freigabevorrichtung so ausgebildet, dass ein Knöchel-Drehmoment, das oberhalb des Drehmoment-Schwellenwerts liegt, das Fußteil in eine Dorsalextensions-Stellung bewegt und dass das Fußteil während einer Schwungphase, in der kein externes Drehmoment an der Prothese anliegt, zumindest im Wesentlichen in der Dorsalextensions-Stellung verbleibt. Geht der Patient mit dieser Prothese, so kommt es während der terminalen Standphase im Gangzyklus zur Dorsalextension des Fußteils relativ zum Unterschenkel-Anschlussteil. In der nachfolgenden Schwungphase ändert sich die Drehstellung des Fußteils relativ zum Unterschenkel-Anschlussteil nicht oder nicht wesentlich.

Unter dem Merkmal, dass sich die Drehstellung nicht im Wesentlichen ändert, wird dabei verstanden, dass während der Schwungphase höchstens ein Drittel des Drehwegs aus der Dorsalextensions- Stellung zurückgelegt wird. Ein besonders großer Abstand zwischen der Fußspitze und dem Boden und damit eine besonders geringe Wahrscheinlichkeit des Stolperns wird dann erreicht, wenn sich das Fußteil in der Schwungphase besonders wenig relativ zum Unterschenkel-Anschlussteil bewegt.

Vorzugsweise umfasst die Freigabevorrichtung (a) einen Hydraulikzylinder, der eine Kolbenstange und ein Zylindergehäuse besitzt und dessen eines Ende mit dem Fußteil und dessen anderes Ende mit dem Unterschenkel-Anschlussteil verbunden ist, und (b) eine Hemmvorrichtung, die in eine Schließstellung bringbar ist, in der sie eine Bewegung der Kolbenstange unterbindet und in eine Öffnungsstellung bringbar ist, in der sie die Bewegung der Kolbenstange ermöglicht. Die Freigabevorrichtung enthält ein Hydraulikfluid, insbesondere eine Hydraulikflüssigkeit. Vorzugsweise weist die Hemmvorrichtung eine Rückschlagarmatur auf.

Gemäß einer bevorzugten Ausführungsform besitzt die Hemmvorrichtung eine Rückschlagarmatur, insbesondere ein ein Überdruck-Rückschlagventil und/oder ein Folgeventil, die in einer Schließstellung eine zu einer Dorsalextensionsbewegung und eine zu einer Plantarflexionsbewegung des Fußteils führende Bewegung der Kolbenstange unterbindet, in einer Öffnungsstellung die Dorsalextensionsbewegung der Kolbenstange ermöglicht und einen Öffnungsdruck pₒₚₑₙ besitzt, bei dem die Rückschlagarmatur aus der Schließstellung in die Öffnungsstellung kommt. Eine solche Prothese ist einfach aufgebaut und zudem leise im Betrieb.

Gemäß einer bevorzugten Ausführungsform ist die Rückschlagarmatur so ausgebildet, dass der Drehmoment-Schwellenwert anhand eines Öffnungsdrucks der Rückschlagarmatur, insbesondere des Überdruck-Rückschlagventils, bei dem die Rückschlagarmatur aus der Schließstellung in die Öffnungsstellung kommt, einstellbar ist. In anderen Worten kann die Rückschlagarmatur eine Öffnungsdruck-Einstellvorrichtung zum Einstellen des Öffnungsdrucks aufweisen. Die resultierende Freigabevorrichtung ist einfach und robust. Vorteilhaft daran ist zudem, dass der Drehmoment-Schwellenwert leicht veränderbar ist.

Unter einer Rückschlagarmatur wird eine Vorrichtung verstanden, die den Durchfluss des Hydraulikfluids in genau eine Durchflussrichtung ermöglicht.

Gemäß einer bevorzugten Ausführungsform umfasst die Hemmvorrichtung ein mechanisches Hemmelement. Das Hemmelement hemmt eine Bewegung des Fußteils, solange das Knöchel-Drehmoment unterhalb des voreingestellten Schwellenwerts liegt. Beispielsweise ist das Hemmelement ausgebildet zum formschlüssigen Zusammenwirken mit einer Ausnehmung. Vorzugsweise ist das Hemmelement in seiner Position so einstellbar, dass die Winkelstellung zwischen Fußteil und Unterschenkel-Anschlussteil, bei der das Hemmelement hemmt, einstellbar ist. Auf diese Weise kann durch Verstellen des Hemmelements eine Änderung der Absatzhöhe eines Schuhs, der mit der Prothese getragen wird, kompensiert werden.

Günstig ist es, wenn die Freigabevorrichtung eine Hemmstruktur und eine Feder aufweist, wobei die Hemmstruktur so mit dem Hemmelement zusammenwirkt, dass eine Bewegung des Fußteils über die Nullstellung hinaus nur gegen den Widerstand einer Feder möglich ist. Auf diese Weise wird eine mechanisch einfache Fußprothese erhalten, bei der die Stolpergefahr verringert ist.

Gemäß einer bevorzugten Ausführungsform weist die die Freigabevorrichtung ein mechanisches Rückschlagelement, einen Kupplungsring und eine Bremse auf, die so miteinander verbunden sind, dass dann, wenn das Fußteil eine Dorsalextensionsbewegung ausführt, das Rückschlagelement so mit dem Kupplungsring kuppelt, dass die Bremse die Dorsalextensionsbewegung bremst, und dass dann, wenn Fußteil eine Plantarflexionsbewegung ausführt, die Bremse die Plantarflexionsbewegung nicht oder weniger stark als die Dorsalextensionsbewegung bremst. So wird das die Stolpergefahr vermindert und dennoch die rückstellende Plantarflexionsbewegung weniger oder nicht behindert.

Die Prothese hat vorzugsweise eine Neutralstellung, die eine Winkelstellung des Fußteils relativ zum Unterschenkel-Anschlussteil ist und in der die Freigabevorrichtung in der Arretierstellung ist. Diese Neutralstellung entspricht der Stellung, die das Fußteil einnimmt, wenn ein Patient, der die Prothese trägt, steht.

Vorzugsweise ist das Fußteil so zum Unterschenkel-Anschlussteil ausgerichtet, dass in der Neutralstellung ein positives Knöchel-Drehmoment am Fußteil anliegt.

Die Hemmvorrichtung ist vorzugsweise so ausgebildet, dass zumindest in einer Winkelposition des Fußteils, die zwischen der Neutralstellung und einer maximal dorsalextendierten Stellung liegt, eine Plantarflexionsbewegung möglich ist. Die Freigabevorrichtung ist vorzugsweise dazu ausgebildet, dass für diese Plantarflexionsbewegung ein Knöchel-Drehmoment notwendig ist, das betragsmäßig höchstens ein Fünftel, insbesondere höchstens ein Zehntel, des Dorsalextensions-Schwellenwerts gemäß Merkmal (e) von Anspruch 1 entspricht.

Günstig ist es, wenn die Hemmvorrichtung eine zweite Rückschlagarmatur aufweist, die in einer Schließstellung eine Plantarflexionsbewegung des Fußteils unterbindet, in einer Öffnungsstellung eine Plantarflexionsbewegung des Fußteils ermöglicht und einen Plantarflexions-Öffnungsdruck besitzt, bei dem die zweite Rückschlagarmatur aus der Schließstellung in die Öffnungsstellung kommt.

Die zweite Rückschlagarmatur ist vorzugsweise der ersten Rückschlagarmatur entgegenwirkend geschaltet. Hierunter ist zu verstehen, dass Hydraulikfluid entweder durch die erste Rückschlagarmatur oder aber durch die zweite Rückschlagarmatur fließen kann, nicht aber durch beide gleichzeitig.

Gemäß einer bevorzugten Ausführungsform ist der Hydraulikzylinder doppelwirkend ausgebildet und so mit der Hemmvorrichtung verbunden, dass eine Dorsal-Extensionsbewegung des Fußteils einen Strom von Hydraulikfluid nur durch die erste Rückschlagarmatur bewirkt und eine Plantarflexionsbewegung des Fußteils einen Strom von Hydraulikfluid nur die zweite Rückschlagarmatur bewirkt. So wird eine einfach aufgebaute Prothese erhalten.

Alternativ weist die Freigabevorrichtung einen zweiten Hydraulikzylinder auf, der eine zweite Kolbenstange und ein zweites Zylindergehäuse besitzt, und dessen eines Ende mit dem Fußteil und dessen anderes Ende mit dem Unterschenkel-Anschlussteil verbunden ist, wobei der zweite Hydraulikzylinder so mit der Hemmvorrichtung und dem ersten Hydraulikzylinder verbunden ist, dass eine Dorsalextensionsbewegung des Fußteils einen Strom von Hydraulikfluid aus dem zweiten Hydraulikzylinder durch die erste Rückschlagarmatur und in den ersten Hydraulikzylinder bewirkt und dass eine Plantarflexionsbewegung des Fußteils einen Strom von Hydraulikfluid aus dem ersten Hydraulikzylinder durch die zweite Rückschlagarmatur und in den zweiten Hydraulikzylinder bewirkt. Auf diese Weise ergibt sich beim Bewegen des Fußteils eine Drehung um eine Drehachse, die zwischen den Hydraulikzylindern liegt, ein physisches Drehgelenk ist entbehrlich.

Vorzugsweise besitzt die Freigabevorrichtung eine magnetorheologische Flüssigkeit und/oder eine elektrorheologische Flüssigkeit. Beispielsweise besitzt der Hydraulikzylinder, in dem die magnetorheologische Flüssigkeit und/oder eine elektrorheologische Flüssigkeit angeordnet ist, einen Dauermagneten, der die Flüssigkeit in ihrem Zustand hoher Viskosität hält, so dass eine Bewegung des Kolbens im Hydraulikzylinder unterbunden wird, bis eine vorgegebene Kraft auf den Kolben überschritten wird. Ist die Kraft auf den Kolben überschritten, bewegt sich der Kolben gedämpft weiter.

Gemäß einer bevorzugten Ausführungsform umfasst die Freigabevorrichtung eine Dämpfeinrichtung, mittels der eine Dorsalextensionsbewegung und/oder eine Plantarflexionsbewegung des Verbindungselements dämpfbar ist. Unter dem Dämpfen wird dabei verstanden, dass dem Knöchel-Drehmoment, das die Dorsalextensionsbewegung des Fußteils relativ zum Unterschenkel-Anschlussteil bewirkt, eine Rückstellkraft entgegengesetzt wird. Da die Dämpfeinrichtung eine Rückstellkraft aufbringt, wirkt die Dämpfeinrichtung als Zeitverzögerungsglied. Es ist möglich, dass die Dämpfeinrichtung zwei oder mehr Dämpfelemente aufweist, beispielsweise Drosseln.

Vorzugsweise ist die Dämpfeinrichtung zum zumindest überwiegend dissipativen Dämpfen ausgebildet, das heißt, dass zumindest 50% der Energie, die in die Dämpfeinrichtung eingeleitet wird, in Wärme umgewandelt wird. Besitzt die Freigabevorrichtung einen Hydraulikzylinder, kann die Dämpfeinrichtung beispielsweise eine Drossel umfassen. Besonders günstig ist es, wenn die Dämpfeinrichtung eine veränderbare Dämpfung ausweist. Umfasst die Dämpfeinrichtung eine Drossel, so handelt es sich vorzugsweise um eine einstellbare Drossel.

Gemäß einer bevorzugten Ausführungsform ist das Fußteil relativ zum Unterschenkel-Anschlussteil in eine Neutralstellung, in der eine die Prothese tragende Person steht, und eine Dorsalextensions-Stellung, in der eine Fußspitze des Fußteils mit dem Unterschenkel-Anschlussteil einen kleineren Winkel bildet als in Neutralstellung, bringbar, wobei die Prothese eine mechanische Vorspannvorrichtung aufweist, mittels der das Fußteil in die Dorsalextensions-Stellung vorgespannt ist. Diese Vorspann-Vorrichtung kann beispielsweise eine Feder aufweisen. Die Vorspann-Vorrichtung ist insbesondere so aufgebaut, dass sie das Fußteil nicht bewegen kann, solange die Freigabevorrichtung in der Arretierstellung ist. Kommt die Freigabevorrichtung dann in ihre Freigabestellung, unterstützt die Vorspannvorrichtung die Dorsalextensionsbewegung des Fußteils und stellt so sicher, dass die Fußspitze stets einen großen Abstand zum Boden hat.

Vorzugsweise ist die Dämpfeinrichtung eingerichtet zum Dämpfen (auch) der Plantarextensionsbewegung des Fußteils. Günstig ist es, wenn die Dämpfung der Plantarextensionsbewegung kleiner ist als die Dämpfung der Dorsalextensionsbewegung. Das führt dazu, dass beim Fersenkontakt zu Beginn des Gangzyklus das Fußteil schnell in seine Neutralstellung kommt. Das fördert einen flüssigen Bewegungsablauf.

Vorzugsweise ist der Drehmoment-Schwellenwert so gewählt, dass die Freigabevorrichtung beim Stehen stets in der Arretierstellung ist. Der Drehmoment-Schwellenwert hängt beispielsweise vom Körpergewicht des Patienten ab und wird bei der Einrichtung der Prothese durch sukzessives Ändern des Drehmoment-Schwellenwerts und Probegehen ermittelt.

Vorzugweise handelt es sich bei der Prothese um einen Prothesenfuß, das heißt, dass sie kein künstliches Kniegelenk umfasst. In diesem Fall ist das passive Umschalten von der Arretierstellung in die Freigabestellung besonders vorteilhaft, da dann keine Messwerte zur Verfügung stehen, anhand derer zusätzlich auf den Zeitpunkt im Gangzyklus geschlossen werden kann.

Günstig ist es, wenn die Prothese ausgebildet ist zum Ausführen einer Dorsalextensionsbewegung von zumindest 3°. So wird ein hinreichend großer Bodenabstand erreicht. Vorteilhaft ist zudem, wenn die Dorsalextensionsbewegung maximal 6° beträgt. Das verhindert zu großen Energieverlust beim Gehen und ein zu tiefes Einknicken des Fußes.

Unter der zum Knöchel-Drehmoment im Verbindungselement korrelierenden Größe wird auch der Angriffspunkt der Kraft im Fußteil verstanden. Wenn der Patient auf dem Vorfuß abrollt, verschiebt sich der Angriffspunkt der (Gewichts- )Kraft zum Vorfuß hin. Das ist beim Gesunden über das Anwinkeln des Zehenbereichs spürbar.

Die Erfindung löst das Problem zudem durch einen Adapater, der ausgebildet ist zum Einbau in eine Prothese, die ein Unterschenkel-Anschlussteil, ein Fußteil und ein Verbindungselement mit Gelenkfunktion, das das Unterschenkel-Anschlussteil mit dem Fußteil verbindet, besitzt. Der Adapter weist eine Freigabevorrichtung auf, mittels der das Fußteil in eine Arretierstellung, in der das Fußteil und das Unterschenkel-Anschlussteil drehstarr miteinander verbunden sind, und in eine Freigabestellung, in der das Fußteil relativ zum Unterschenkel-Anschlussteil drehbar ist, bringbar ist, wobei die Freigabevorrichtung ausgebildet ist zum Umschalten von der Arretierstellung in die Freigabestellung, wenn eine zu einem Knöchel-Drehmoment im Verbindungselement korrelierende Größe oberhalb eines voreingestellten Schwellenwerts liegt. Ein derartiger Adapter kann auch parallel zu einer etwaig vorhandenen Hydraulik und/oder mechatronischen Steuerung der Prothese eingesetzt werden.

Im Folgenden wird die Erfindung anhand der beigefügten Zeichnungen näher erläutert. Dabei zeigt
- Figur 1: eine erste Ausführungsform einer erfindungsgemäßen Prothese gemäß einer ersten Ausführungsform,
- Figur 2: in den Teilfiguren 2a, 2b und 2c eine mögliche Ausführungsform einer Freigabevorrichtung und in der Teilfigur 2d den Verlauf des Knöchel-Drehmoments im Ablauf des Gangzyklus,
- Figur 3: mit den Teilfiguren 3a und 3b eine zweite Ausführungsform einer erfindungsgemäßen Prothese,
- Figur 4: in der Teilfigur 4a eine dritte Ausführungsform einer erfindungsgemäßen Prothese und in den Teilfiguren 4b und 4c ein Schaltbild der zugehörigen Freigabevorrichtung,
- Figur 5: eine vierte Ausführungsform einer erfindungsgemäßen Prothese und
- Figur 6: eine fünfte Ausführungsform einer erfindungsgemäßen Prothese.
- Figur 7: zeigt eine sechste Ausführungsform einer erfindungsgemäßen Prothese, bei der anhand des Körperschwerpunkts umgeschaltet wird, und
- Figur 8: den Verlauf der Kraft des Knöchel-Drehmoments im Ablauf des Gangzyklus.
- Figur 9: zeigt die Freigabevorrichtung einer siebten Ausführungsform einer erfindungsgemäßen Prothese, bei der die Rückschlagarmatur ein Folgeventil aufweist,
- Figur 10: zeigt die Freigabevorrichtung einer achten Ausführungsform einer erfindungsgemäßen Prothese, bei der die Freigabevorrichtung zwei Hydraulikzylinder hat, und
- Figur 11: zeigt die Freigabevorrichtung einer neunten Ausführungsform einer erfindungsgemäßen Prothese, bei der die Hemmvorrichtung ein mechanisches Hemmelement hat.
- Figur 12: zeigt eine weitere Ausführungsform einer erfindungsgemäßen Prothese, bei der das mechanische Hemmelement 88 der Freigabevorrichtung 16 als Gleitelement ausgebildet ist, das mit einer Hemmstruktur 92 zusammenwirkt.

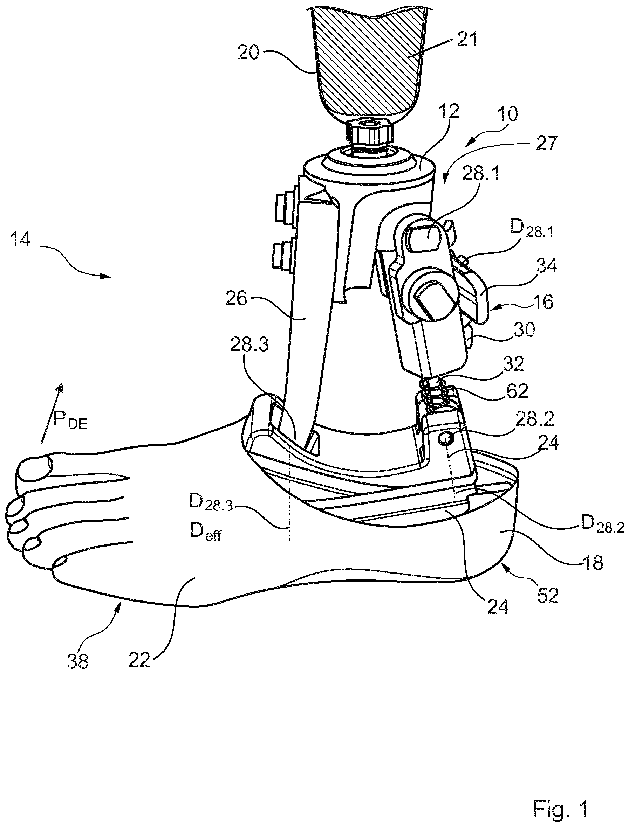

Figur 1 zeigt eine erfindungsgemäße Prothese 10 mit einem Unterschenkel-Anschlussteil 12, einem Verbindungselement 14, einer Freigabevorrichtung 16 sowie einem Fußteil 18. Das Fußteil 18 ist über das Verbindungselement 14 mit dem Unterschenkel-Anschlussteil 12 verbunden. Das Unterschenkel-Anschlussteil 12 ist zum Verbinden mit einer schematisch eingezeichneten Unterschenkelaufnahme 20 ausgebildet.

Das Fußteil 18 umfasst eine Fußkosmetik 22, die der Prothese 10 ein natürliches Aussehen verleiht. Das Fußteil 18 weist zudem eine Standplatte 24 auf, an der die Fußkosmetik 22 sowie das Verbindungselement 14 befestigt sind.

Das Verbindungselement 14 umfasst im vorliegenden Fall einen starren Arm 26, der mittig am Fußteil 18 befestigt ist. Die Freigabevorrichtung 16 bildet einen zweiten, längenveränderlichen Arm 27, der an einem Fersenabschnitt der Prothese 10 am Fußteil 18 befestigt ist.

Figur 1 zeigt, dass das Verbindungselement 14 drei Teil-Gelenke 28.1, 28.2, 28.3 umfasst, die alle drei Drehgelenke sind und jeweilige Drehachsen D_{28.1}, D_{28.2} und D_{28.3} aufweisen. Bei Bewegung der Prothese 10 dreht sich das Fußteil 18 relativ zum Unterschenkel-Anschlussteil 12 um eine Drehachse D_{eff}.

Die Freigabevorrichtung 16 umfasst einen Hydraulikzylinder 30, der eine Kolbenstange 32 und ein Zylindergehäuse 34 besitzt. Der Hydraulikzylinder 30 ist mit seinem einen Ende im Teil-Gelenk 28.1 mit dem Unterschenkel-Anschlussteil 12 verbunden. Mit dem anderen Ende ist der Hydraulikzylinder 30 über das Teil-Gelenk 28.2 mit dem Fußteil 18 verbunden.

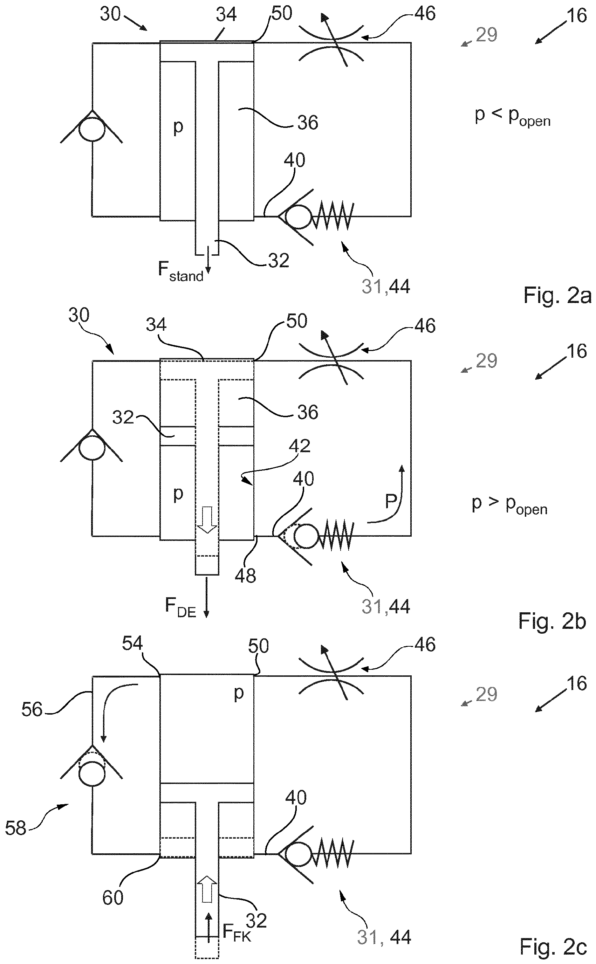

Figur 2a zeigt schematisch die Freigabevorrichtung 16. Die Freigabevorrichtung 16 weist eine Hemmvorrichtung 29 auf, die mit dem Hydraulikzylinder 30 verbunden ist. Es ist zu erkennen, dass die Kolbenstange 32 des Hydraulikzylinders 30 in einem Zylindergehäuse 34 läuft, das mit einem Fluid 36, insbesondere einer Hydraulikflüssigkeit, gefüllt ist. Befindet sich die Prothese 10 in ihrer Standposition, die in Figur 1 gezeigt ist, so wirkt eine Standkraft F_{stand} auf die Kolbenstange 32, die in diesem Zustand maximal in das Zylindergehäuse 34 eingefahren ist. Die Freigabevorrichtung 16 ist in diesem Zustand steif. Die Hemmvorrichtung 29 und damit die Freigabevorrichtung 16 sind damit in einer Schließstellung, in der die Kolbenstange 32 sich nicht bewegen kann.

In der mittleren Standphase entsteht eine zunehmende Kraft auf einen Ballenbereich 38 (vgl. Figur 1) der Prothese 10. Daraus resultiert ein Knöchel- Drehmoment M um die Drehachse D_{28.3}. Das führt dazu, dass die Kraft F auf die Kolbenstange 32 abnimmt und schließlich negativ wird und die Kolbenstange 32 auf Zug belastet wird. Dadurch steigt ein Druck p im Zylindergehäuse 34 an.

Die Hemmvorrichtung 29 besitzt eine Ableitung 40, die mit einem Innenraum 42 des Zylindergehäuses 34 in Verbindung steht und eine Rückschlagarmatur 31, die im vorliegenden Fall durch ein Überdruck-Rückschlagventil 44 gebildet ist. Die Ableitung 40 kann zudem eine einstellbare Drossel 46 umfassen und endet im Zylindergehäuse 34 in einer Ausströmöffnung 50. Eine Einströmöffnung 48 der Ableitung 40 ist von einer Ausströmöffnung 50 durch die Kolbenstange 32 getrennt.

Beim Fortschreiten im Gangzyklus steigt das Knöchel-Drehmoment *̅M̅*̅, das das Fußteil 18 und das Unterschenkel-Anschlussteil 12 auf das Verbindungselement 14 aufbringen, kontinuierlich an. Dadurch sinkt die Kraft F, die auf die Kolbenstange 32 wirkt, bis sie zunächst null wird. Danach wirkt eine negative Kraft F_{DE}, also eine Zugkraft auf die Kolbenstange 32.

Figur 2b zeigt, dass dadurch der Druck p an der Einströmöffnung 48 ansteigt. Sobald der Druck p größer ist als ein Öffnungsdruck pₒₚₑₙ des Überdruck-Rückschlagventils 44, öffnet dieses und Fluid 36 strömt wie durch den Pfeil P angedeutet auf die Ausströmöffnung 50 zu. in anderen Worten ist die Hemmvorrichtung 29 in der Öffnungsstellung, Dadurch bewegt sich die Kolbenstange 32 im Zylindergehäuse 34 und es kommt zu einer Dorsalextensionsbewegung, die in Figur 1 mit dem Pfeil P_{DE} gekennzeichnet ist. Durch die einstellbare Drossel 46 wird der Fluss des Fluids 36 durch die Ableitung 40 so gedämpft, dass ein für den Patienten angenehmer Bewegungsablauf erhalten wird. In anderen Worten erfolgt eine zusätzliche Bewegung des Fußteils, die mehr Bodenfreiheit erzeugt und die in der Schwungphase erhalten bleibt.

Figur 2c zeigt gestrichelt die Kolbenstange 32 in ihrer Stellung am Ende der Standphase.

Es ist möglich und stellt eine bevorzugte Ausführungsform dar, dass die Prothese eine Rückstellfeder aufweist, die das Fußteil in der Schwungphase in seine Neutralstellung, also die Stellung im Stand, zurückbringt.

In der Schwungphase hat das Fußteil 18 keinen Kontakt mehr mit dem Boden. Besitzt die Prothese - wie in der vorliegenden Ausführungsform - keine Rückstellfeder, bewegt sich die Kolbenstange 32 nicht relativ zum Zylindergehäuse 34.

Nach Ende der Schwungphase beginnt die Standphase, wenn ein Fersenabschnitt 52 der Prothese 10 auf den Boden auftrifft. Dadurch wirkt eine Druckkraft F_{FK} auf die Kolbenstange 32. Figur 2c lässt erkennen, dass der Druck p an der Ausströmöffnung 50 ansteigt, das Fluid 36 kann jedoch durch die Ableitung 40 nicht wegströmen, da das Überdruck-Rückschlagventil 44 sperrt. Das Fluid 36 fließt durch eine Zuströmöffnung 54 einer Rückleitung 56 durch eine zweite Rückschlagarmatur 46, die im vorliegenden Fall durch ein Rückschlagventil gebildet ist, zu einer Rückströmöffnung 60, so dass sich die Kolbenstange 32 in ihre Ausgangslage zurückbewegen kann, die in Figur 2a gezeigt ist.

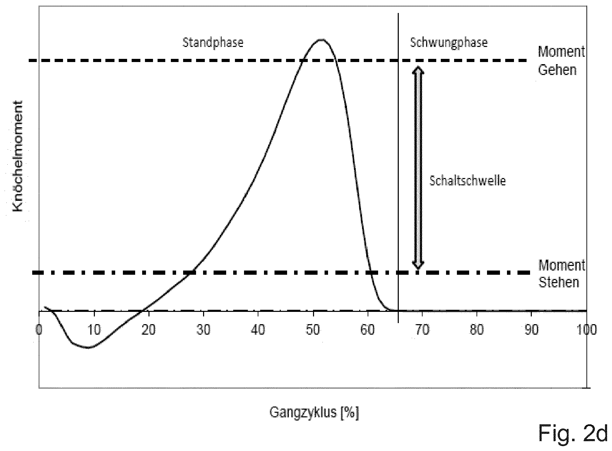

Figur 2d zeigt den Verlauf des Knöchel-Drehmoments M in beliebigen Einheiten vom Fortschritt im Gangzyklus. Es ist zu erkennen, dass als Schwellenwert jedes Knöchel-Drehmoment M gewählt werden kann, dass größer ist als das Knöchel-Drehmoment M im Stehen. Zum Einstellen des Schwellenwerts wird beispielsweise das Knöchel-Drehmoment M im Stand erfasst und danach das Knöchel-Drehmoment M größer eingestellt. Danach wird ermittelt, ob dieser Schwellenwert hinreichend klein ist, sodass die Freigabestellung im Gangzyklus erreicht wird.

Figur 1 zeigt, dass die Prothese 10 eine Vorspannvorrichtung 62 aufweisen kann, die im vorliegenden Fall durch die Druckfeder gebildet ist. Die Vorspannvorrichtung 62 übt eine Vorspannkraft auf das Fußteil 18 aus, die das Fußteil 18 in eine Dorsalextensions-Stellung vorspannt.

Gemäß einer alternativen Ausführungsform ist die Vorspannvorrichtung 62 durch eine Zugfeder gebildet, die das Fußteil 18 in eine Plantarextensions-Stellung vorspannt.

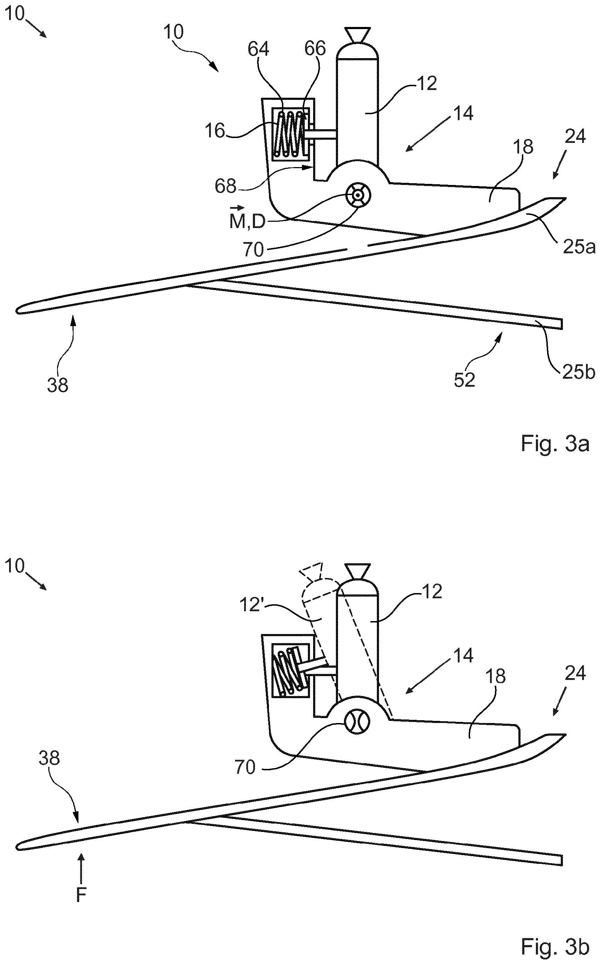

Figur 3 zeigt mit seinen Teilfiguren 3a und 3b eine zweite Ausführungsform einer erfindungsgemäßen Prothese 10. Die Standplatte 24 umfasst zwei Federelemente 25a, 25b, beispielsweise aus Karbonfasern, die aneinander befestigt sind. Die Freigabevorrichtung 16 ist seriell zu den Federelementen 25a, 25b und zugbelastet angeordnet. Die Freigabevorrichtung 16 umfasst eine gefangene Druckfeder 64, die einen Teller 66 gegen einen Anschlag 68 vorspannt. Der Anschlag 68 ist im vorliegenden Fall durch die Wand eines Käfigs der Druckfeder 64 gebildet. Figur 3a zeigt den Zustand im Stand.

Figur 3b zeigt den Zustand am Ende der Standphase. Durch eine Kraft F auf den Ballenbereich 38 steigt das Knöchel-Drehmoment M so weit an, dass der Teller 66 sich vom Anschlag 68 löst und gegen die Kraft der Druckfeder 64 diese zusammendrückt. Dadurch kommt das Fußteil 18 in eine dorsalextendierte Stellung. In Figur 3b ist dies durch das gestrichelte Unterschenkel-Anschlussteil 12' gezeigt.

Die Prothese 10 besitzt eine Dämpfeinrichtung 70, die eine Rückstellbewegung, die zumindest auch von der Druckfeder 64 bewirkt wird, dämpft.

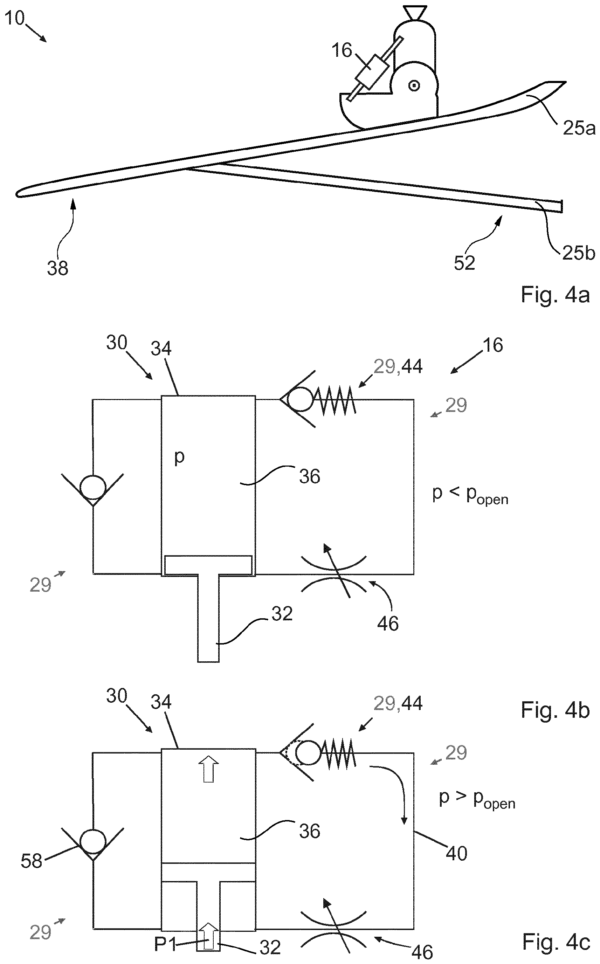

Figur 4a zeigt eine Prothese, bei der die schematisch eingezeichnete Freigabevorrichtung 16 seriell zu den Federelementen 25a, 25b angeordnet ist. Die Freigabevorrichtung16 ist druckbelastet ausgeführt.

Figur 4b zeigt die Stellung der Freigabevorrichtung 16 in der Standphase. Die Kolbenstange 32 ist am Anschlag im Zylindergehäuse 34, der Druck p ist kleiner als der Öffnungsdruck pₒₚₑₙ und die Freigabevorrichtung 16 ist steif. Die Hemmvorrichtung 29 ist in der Schließstellung.

Figur 4c zeigt die Bewegung der Kolbenstange 32, wenn der Druck p den Öffnungsdruck pₒₚₑₙ übersteigt. Durch die Ableitung 40 und die Drossel 46 fließt das Fluid 36. Die Hemmvorrichtung 29 ist in der Öffnungsstellung.

Beim Fersenauftritt bewegt sich die Kolbenstange 32 entgegen dem Pfeil P1, in Figur 4c also nach unten, und das Rückschlagventil 58 öffnet, wohingegen das Überdruck-Rückschlagventil 44 sperrt.

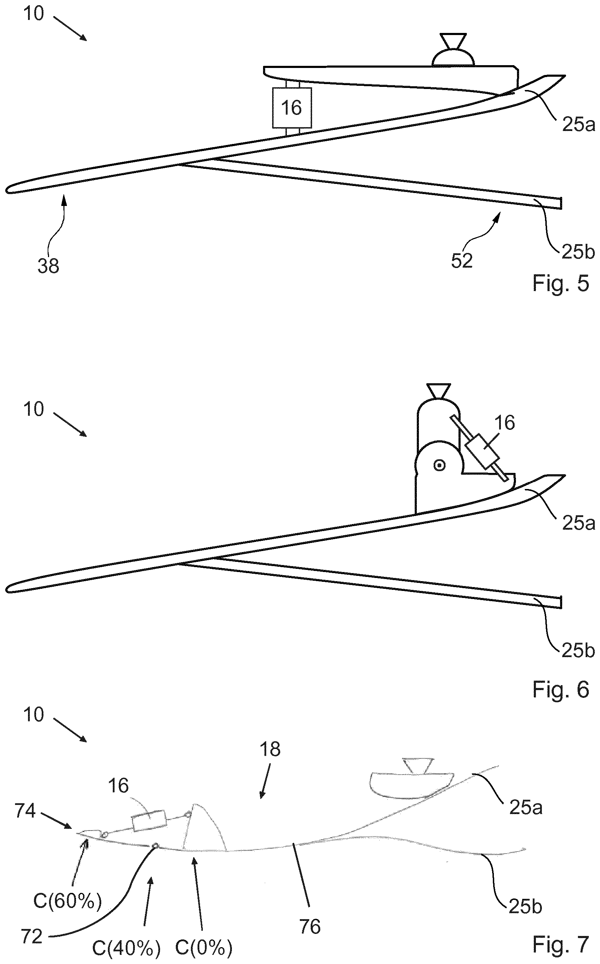

Figur 5 zeigt eine Prothese 10, bei der die schematisch eingezeichnete Freigabevorrichtung 16 druckbelastet und parallel zu den Federelementen 25a, 25b wirkend angeordnet ist.

Figur 6 zeigt eine Prothese 10, bei der die schematisch eingezeichnete Freigabevorrichtung 16 zugbelastet und seriell zu den Federelementen 25a, 25b wirkend angeordnet ist.

Figur 7 zeigt eine Prothese 10, bei der die Freigabevorrichtung 16 ausgebildet ist zum Umschalten von der Arretierstellung in die Freigabestellung, wenn die Lage der Projektion des Körperschwerpunkts auf das Fußteil einen voreingestellten Schwellenwert überschreitet. Im Gangzyklus bewegt sich der Körperschwerpunkt C in Abhängigkeit vom Fortschritt im Gangzyklus in Prozenten.

Es ist zu erkennen, dass das Fußteil 18 ein Vorfußgelenk 72 aufweist, das eine Fußspitze 74 und ein Mittelfußteil 76 miteinander verbindet. Die Freigabevorrichtung 16 überbrückt das Vorfußgelenk 72 und ist einerseits an der Fußspitze 74 und andererseits am Mittelfußteil 76 befestigt.

Zu Beginn des Gangzyklus liegt die Projektion C des Körperschwerpunkts im Mittelfußteil 76 und wandert auf das Fußteil 18 zu. Sobald die Projektion C des Körperschwerpunkts das Vorfußgelenk 72 erreicht hat, im vorliegenden Fall bei 40% des Gangzyklus, wird die Freigabevorrichtung 16 auf Druck belastet. Nach Überschreiten des Öffnungsdrucks kommt die Freigabevorrichtung in die Freigabestellung und die Fußspitze 74 bewegt sich auf das Mittelfußteil 76 zu. Das bleibt in der Schwungphase erhalten und verschafft mehr Bodenfreiheit.

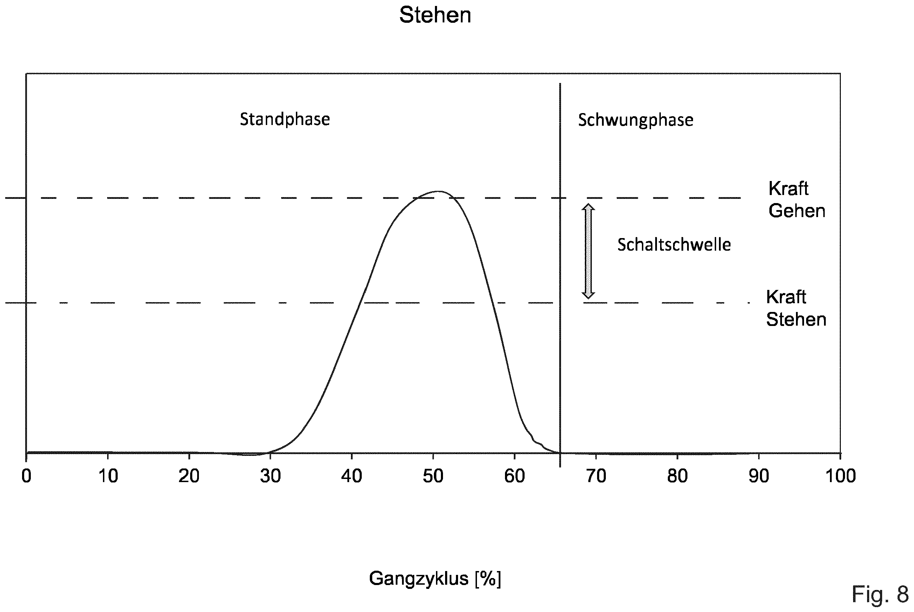

Figur 8 zeigt den Verlauf der Kraft K in beliebigen Einheiten vom Fortschritt im Gangzyklus. Es ist zu erkennen, dass als Schwellenwert jede Kraft K gewählt werden kann, welche größer ist als die Kraft K im Stehen. Zum Einstellen des Schwellenwerts wird beispielsweise die Kraft K im Stand erfasst und danach die Kraft K größer eingestellt. Danach wird ermittelt, ob dieser Schwellenwert hinreichend klein ist, sodass die Freigabestellung im Gangzyklus erreicht wird. Es wird vorzugsweise zudem ermittelt, ob der Schwellenwert hinreichend groß ist, um eine stabiles Stehen zu ermöglichen.

Als alternative Ausführungsform in Figur 5 könnte die Freigabeeinrichtung 16 ein über den Kraftverlauf aus Figur 8 gesteuertes Element sein, welches bei Überschreiten eines einstellbaren Schwellenwerts der Kraft K schaltet. Die Kraft K ist in dieser Ausführungsform eine resultierende Kraft aus der Gewichtskraft des Patienten.

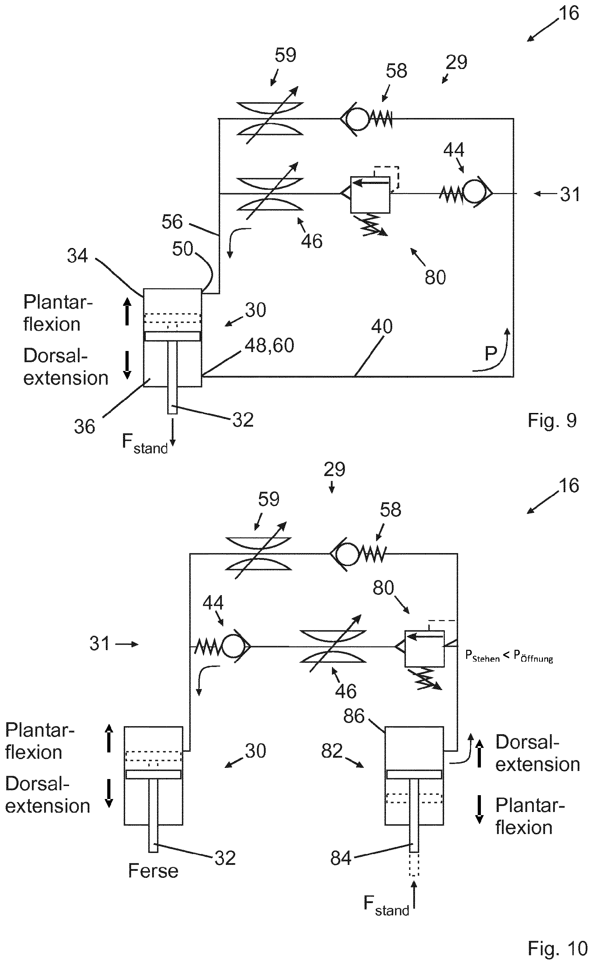

Figur 9 zeigt eine Freigabevorrichtung 16 für eine erfindungsgemäße Prothese, bei der die Rückschlagarmatur 31 neben einem Rückschlagventil 44 ein Folgeventil 80 aufweist. Figur 9 zeigt zudem, dass die Freigabevorrichtung 16 eine Dämpfeinrichtung 59 aufweist.

Führt das Fußteil eine Dorsalextensionsbewegung aus, so fließt das Hydraulikfluid, wenn das Knöchel-Drehmoment den Dorsalextension-Schwellenwert überschreitet, aus der Öffnung 48, durch die erste Rückschlagarmatur 31 und durch die Öffnung 50 zurück in den Hydraulikzylinder 34 in einen Raum, der durch den Kolben des Hydraulikzylinders 34 von dem Raum getrennt ist, der mit der Öffnung 48 verbunden ist. Führt das Fußteil eine Plantarflexionsbewegung aus, so strömt das Hydraulikfluid aus der Öffnung 50, durch die zweite Rückschlagarmatur 58 und durch die Öffnung 48 zurück in den Hydraulikzylinder 34.

Figur 10 zeigt eine Ausführungsform, bei der die Freigabevorrichtung 16 einen zweiten Hydraulikzylinder 82 aufweist, der eine zweite Kolbenstange 84 und ein zweites Zylindergehäuse 86 besitzt und der so mit dem ersten Zylinder 30 verbunden ist, dass ein Einschieben der zweiten Kolbenstange 84 in das zweite Zylindergehäuse 86 dazu führt, dass die erste Kolbenstange 32 aus dem ersten Zylindergehäuse 34 herausgedrückt wird.

Der zweite Hydraulikzylinder 82 ist vorzugsweise so angeordnet, dass er den starren Arm 26 (vgl. Fig. 1) ersetzt. In diesem Fall ist die zweite Kolbenstange 84 im Teilgelenk 28.3 mit dem Fußteil 18, insbesondere der Standplatte 24, verbunden.

Führt das Fußteil eine Dorsalextensionsbewegung aus, so fließt das Hydraulikfluid, wenn das Knöchel-Drehmoment den Dorsalextension-Schwellenwert überschreitet, aus dem zweiten Hydraulikzylinder 82, nur durch die erste Rückschlagarmatur 31 in den ersten Hydraulikzylinder 30. Führt das Fußteil eine Plantarflexionsbewegung aus, so strömt das Hydraulikfluid aus dem ersten Hydraulikzylinder 30, nur durch die zweite Rückschlagarmatur 58 in den zweiten Hydraulikzylinder 34.

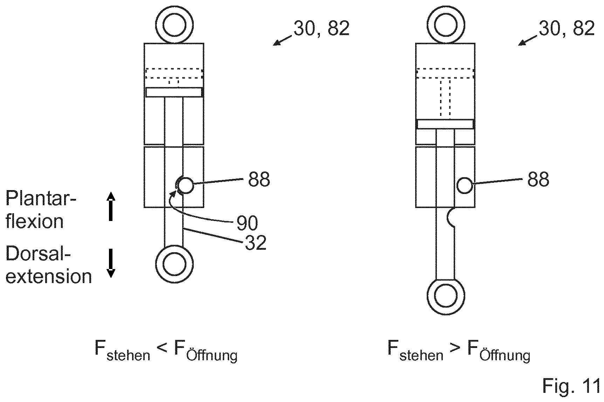

Figur 11 zeigt eine alternative Ausführungsform für den ersten Hydraulikzylinder 30 oder, soweit vorhanden, den zweiten Hydraulikzylinder 82. Es ist zu erkennen, dass der Hydraulikzylinder 30 ein Hemmelement 88 aufweist, das wie im vorliegenden Fall durch einen Stift oder einen Bolzen gebildet sein kann. Das Element 88 wirkt formschlüssig mit einer Ausnehmung 90 zusammen und hemmt damit eine Bewegung der Kolbenstange 32. Sofern die Prothese lediglich einen Hydraulikzylinder umfasst, bewirkt das Element, dass das Fußteil 18 (vgl. Fig. 1) sich relativ zur Unterschenkelaufnahme 20 nicht verschwenken kann, solange das Drehmoment auf das Fußteil hinreichend klein ist.

Das rechte Teilbild zeigt die Situation, in der das Knöchel-Drehmoment den Schwellenwert überschritten hat, was dazu führt, dass das Hemmelement 88 aus der Ausnehmung 90 herausgerutscht ist. Die Kolbenstange 32 kann sich nun frei bewegen. Es ist günstig, wenn das Hemmelement 88 auf die Ausnehmung 90 vorgespannt ist, beispielweise mittels einer Feder. Alternativ oder zusätzlich ist es möglich, dass das Hemmelement 88 federnd befestigt ist. Die Form des Hemmelements 88 sowie der Ausnehmung 90 und die Kraft, mit der das Hemmelement 88 in die Ausnehmung 90 gedrückt wird, beeinflussen den Schwellenwert, ab dem das Fußteil eine Dorsalextensionsbewegung ausführen kann.

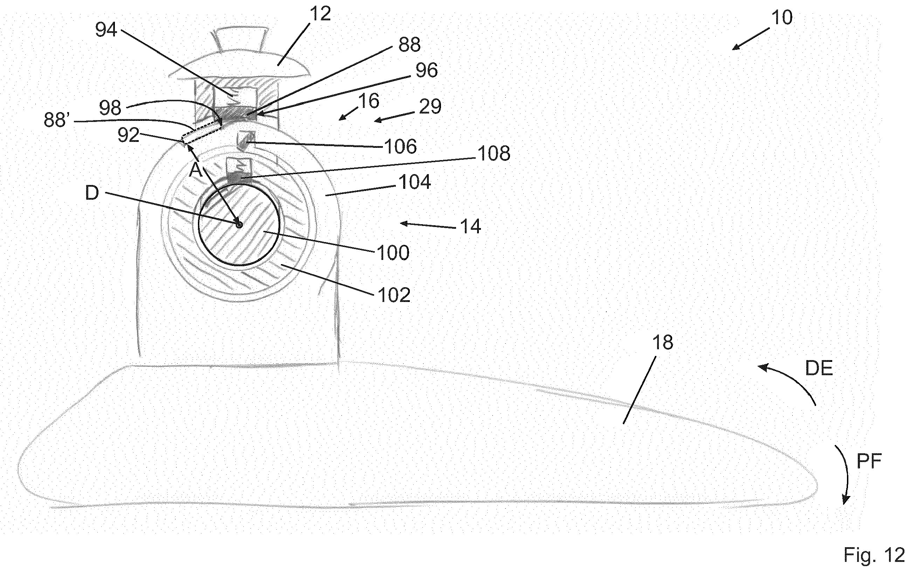

Figur 12 zeigt eine weitere Ausführungsform einer erfindungsgemäßen Prothese, bei der das mechanische Hemmelement 88 der Freigabevorrichtung 16 als Gleitelement ausgebildet ist, das mit einer Hemmstruktur 92 zusammenwirkt. Die Hemmstruktur ist gebildet durch einen Oberflächenverlauf, wobei die Oberfläche einen variierenden Abstand A von der Drehachse D hat.

Ist das Fußteil 18 in seiner Nullstellung, so muss ein Knöcheldrehmoment M am Fußteil 18 anliegen, um das Gleitelement 88 gegen eine Federkraft einer Feder 94 nach radial auswärts zu bewegen. In diese Stellung ist das Gleitelement mit 88' bezeichnet und gestrichelt eingezeichnet.

Solange das Knöcheldrehmoment M unter dem Schwellenwert liegt, liegt das Gleitelement 88 mit einer Vorderkante 96 an einer Steigung 98 der Hemmstruktur an, sodass eine Dorsalextensionsbewegung des Fußteils 18 verhindert wird. Eine Plantarflexionsbewegung hingegen ist weiterhin möglich und wird durch den Druck der Feder 94 sogar unterstützt.

Im vorliegenden Fall besitzt das Fußteil 18 einen Zapfen 100, um den ein Kupplungsring 102 angeordnet ist. Um den Kupplungsring 102 ist eine Umfassungsstruktur 104 des Fußteils 18 angeordnet.

Die Freigabevorrichtung 16 umfasst ein Rückschlagelement 106, das im vorliegenden Fall durch eine einseitig gelagerte Klappe gebildet ist. Das mechanische Rückschlagelement 106 ist so angeordnet, dass eine Plantarflexionsbewegung PF des Fußteils 106 zu einem Durchrutschen des Rückschlagelements relativ zum Kupplungsring 102 führt. Eine Dorsalextensionsbewegung DE des Fußteils 18 führt hingegen dazu, dass das Rückschlagelement 106 reibschlüssig mit dem Kupplungsring 102 koppelt. Daher dreht der Kupplungsring 102 mit.

Der Kupplungsring 102 umfasst eine Bremse 108, die eine Dorsalextensionsbewegung des Fußteils 18 bremst. Die eine Bremse 108, der Kupplungsring 102 und das Rückschlagelement 106 sind so miteinander verbunden, dass dann, wenn das Fußteil 18 eine Dorsalextensionsbewegung ausführt, das Rückschlagelement 106 mit dem Kupplungsring 102 kuppelt und die Bremse die Dorsalextensionsbewegung bremst, Führt hingegen das Fußteil 18 eine Plantarflexionsbewegung aus, bremst die Bremse 108 die Plantarflexionsbewegung nicht oder weniger stark als sie die Dorsalextensionsbewegung.

Es ist möglich und stellt eine bevorzugte Ausführungsform dar, dass die Hemmstruktur 92 relativ zum Fußteil 18 in einer bezüglich der Drehachse D radialen Richtung lösbar befestigt ist. Beispielsweise ist die Hemmstruktur 92 an einem relativ zum Fußteil 18 festlegbar bewegbaren Einstellteil ausgebildet ist. In diesem Fall kann dieses Einstellteil zunächst vom Fußteil 18 gelöst, dann relativ zum Fußteil 18 um die Drehachse D geschwenkt und danach wieder am Fußteil 18 befestigt werden. Auf diese Weise verändert sich die Winkelstellung, in der das Fußteil 18 in der Nullstellung ist. So kann die Nullstellung eingestellt werden, ab der die Dorsalextensionsbewegung stärker gehemmt wird.

**Bezugszeichenliste**

| | | | |
|---|---|---|---|
| 10 | Prothese | 34 | Zylindergehäuse |
| 12 | Unterschenkel-Anschlussteil | 36 | Fluid |
| 14 | Verbindungselement | 38 | Ballenbereich |
| 16 | Freigabevorrichtung | | |
| 18 | Fußteil | 40 | Ableitung |
| | | 42 | Innenraum |
| 20 | Unterschenkelaufnahme | 44 | Überdruck-Rückschlagventil |
| 21 | Unterschenkel | 46 | einstellbare Drossel |
| 22 | Fußkosmetik | 48 | Einströmöffnung |
| 24 | Standplatte | | |
| 26 | starrer Arm | 50 | Ausströmöffnung |
| 27 | zweiter Arm | 52 | Fersenabschnitt |
| 28 | Teil-Gelenk | 54 | Zuströmöffnung |
| 29 | Hemmvorrichtung | 56 | Rückleitung |
| | | 58 | zweite Rückschlagarmatur, Rückschlagventil |
| 30 | Hydraulikzylinder | 59 | Dämpfeinrichtung |
| 31 | Rückschlagarmatur | | |
| 32 | Kolbenstange | 60 | Rückströmöffnung |
| 62 | Vorspannvorrichtung | 98 | Steigung |
| 64 | Druckfeder | | |
| 66 | Teller | 100 | Zapfen |
| 68 | Anschlag | 102 | Kupplungsring |
| | | 104 | Umfassungsstruktur |
| 70 | Dämpfeinrichtung | 106 | Rückschlagelement |
| 72 | Vorfußgelenk | 108 | Bremse |
| 74 | Fußspitze | | |
| 76 | Mittelfußteil | D | Drehachse |
| 78 | Rückschlagelement | D_{eff} | momentane Drehachse |
| | | DF | Plantarextensionsrichtung |
| 80 | Folgeventil | DE | Dorsalextensionsrichtung |
| 82 | zweiter Hydraulikzylinder | | |
| 84 | zweite Kolbenstange | F | Kraft |
| 86 | zweites Zylindergehäuse | F_{stand} | Stand kraft |
| 88 | Hemmelement | M | Knöchel-Drehmoment |
| | | | |
| 90 | Ausnehmung | p_{stand} | Standruck |
| 92 | Hemmstruktur | p | Druck |
| 94 | Feder | pₒₚₑₙ | Öffnungsdruck |
| 96 | Vorderkante | P | Pfeil |

## Patentansprüche

1. Prothese mit
(a) einem Unterschenkel-Anschlussteil (12),
(b) einem Fußteil (18),
(c) einem Verbindungselement (14) mit Gelenkfunktion, das
das Unterschenkel-Anschlussteil (12) mit dem Fußteil (18) verbindet, und
(d) einer Freigabevorrichtung (16), die
- in eine Arretierstellung, in der das Fußteil (18) und das Unterschenkel-Anschlussteil (12) zumindest bezüglich einer Dorsalextensionsbewegung drehstarr miteinander verbunden sind, und
- in eine Freigabestellung, in der das Fußteil (18) relativ zum Unterschenkel-Anschlussteil (12) drehbar ist, bringbar ist,
**dadurch gekennzeichnet, dass**
(e) die Freigabevorrichtung (16) ausgebildet ist zum passiven Umschalten von der Arretierstellung in die Freigabestellung, wenn eine zu einem Knöchel- Drehmoment (M) im Verbindungselement (14) korrelierende Größe oberhalb eines voreingestellten Schwellenwerts liegt.

2. Prothese nach Anspruch 1, **dadurch gekennzeichnet, dass** die Freigabevorrichtung (16) ausgebildet ist zum Umschalten von der Arretierstellung in die Freigabestellung, wenn das Knöchel-Drehmoment (M) oberhalb eines voreingestellten Drehmoment-Schwellenwerts liegt.

3. Prothese nach Anspruch 2, **dadurch gekennzeichnet, dass** die Freigabevorrichtung (16) so ausgebildet ist, dass
- ein Knöchel-Drehmoment (M), das oberhalb des Drehmoment-Schwellenwerts liegt, das Fußteil (18) in eine dorsalextendierte Stellung bewegt und dass
- das Fußteil (18) während einer Schwungphase zumindest im Wesentlichen in der dorsalextendierten Stellung verbleibt.

4. Prothese nach einem der vorstehenden Ansprüche, **dadurch gekennzeichnet, dass** die Freigabevorrichtung (16)
(a) einen Hydraulikzylinder (30), der
- eine Kolbenstange (32) und ein Zylindergehäuse (34) besitzt und
- dessen eine Ende mit dem Fußteil (18) und dessen andere Ende mit dem Unterschenkel-Anschlussteil (12) verbunden ist, und
(b) eine Hemmvorrichtung (29) aufweist, die
- in einer Schließstellung eine zu einer Dorsalextensionsbewegung des Fußteils (18) führende Bewegung der Kolbenstange (32) unterbindet,
- in einer Öffnungsstellung die Bewegung der Kolbenstange (32) ermöglicht.

5. Prothese nach Anspruch 4, **dadurch gekennzeichnet, dass** die Hemmvorrichtung (29) eine Rückschlagarmatur (31), insbesondere ein ein Überdruck-Rückschlagventil (44) und/oder ein Folgeventil (80), umfasst, die
- in einer Schließstellung eine zu einer Dorsalextensionsbewegung des Fußteils (18) führende Bewegung der Kolbenstange (32) unterbindet,
- in einer Öffnungsstellung die Dorsalextensionsbewegung der Kolbenstange (32) ermöglicht und
- einen Öffnungsdruck (pₒₚₑₙ) besitzt, bei dem die Rückschlagarmatur (31) aus der Schließstellung in die Öffnungsstellung kommt.

6. Prothese nach einem der Ansprüche 4 bis 5, **dadurch gekennzeichnet, dass** die Hemmvorrichtung (29) ein mechanisches Hemmelement (88) aufweist.

7. Prothese nach Anspruch 6, **dadurch gekennzeichnet, dass** die Freigabevorrichtung (16) eine Hemmstruktur (92) und eine Feder (94) aufweist, wobei die Hemmstruktur so mit dem Hemmelement (88) zusammenwirkt dass eine Bewegung des Fußteils über die Nullstellung hinaus nur gegen den Widerstand einer Feder (94) möglich ist.

8. Prothese nach Anspruch 6 oder 7, **dadurch gekennzeichnet, dass** die Freigabevorrichtung (16)
- ein mechanisches Rückschlagelement (106),
- einen Kupplungsring (102) und
- eine Bremse (108) aufweist, die so miteinander verbunden sind, dass dann, wenn das Fußteil (18) eine Dorsalextensionsbewegung ausführt, das Rückschlagelement (106) so mit dem Kupplungsring (102) kuppelt, dass die Bremse die Dorsalextensionsbewegung bremst, und dass dann, wenn Fußteil (18) eine Plantarflexionsbewegung ausführt, die Bremse (108) die Plantarflexionsbewegung nicht oder weniger stark als die Dorsalextensionsbewegung bremst.

9. Prothese nach Anspruch 4 bis 6, **dadurch gekennzeichnet, dass** die Hemmvorrichtung (29) eine zweite Rückschlagarmatur (58) aufweist, die
- in einer Schließstellung eine Plantarflexionsbewegung des Fußteils (18) unterbindet und
- in einer Öffnungsstellung eine Plantarflexionsbewegung des Fußteils (18) ermöglicht und
- einen Plantarflexions-Öffnungsdruck (p_{open,flex}) besitzt, bei dem die zweite Rückschlagarmatur (58) aus der Schließstellung in die Öffnungsstellung kommt.

10. Prothese nach Anspruch 9, **dadurch gekennzeichnet, dass** der Hydraulikzylinder (30)
- doppelwirkend ausgebildet ist und
- so mit der Hemmvorrichtung (29) verbunden ist, dass
eine Dorsalextensionsbewegung des Fußteils (18) einen Strom von Hydraulikfluid (36) nur durch die erste Rückschlagarmatur (31) bewirkt und eine Plantarflexionsbewegung des Fußteils einen Strom von Hydraulikfluid (36) nur durch die zweite Rückschlagarmatur (58) bewirkt.

11. Prothese nach Anspruch 9, **dadurch gekennzeichnet, dass** die Freigabevorrichtung (16) einen zweiten Hydraulikzylinder (82) aufweist, der
- eine zweite Kolbenstange (84) und ein zweites Zylindergehäuse (86) besitzt und
- dessen eine Ende mit dem Fußteil (18) und dessen andere Ende mit dem Unterschenkel-Anschlussteil (12) verbunden ist,
- so mit der Hemmvorrichtung (29) und dem ersten Hydraulikzylinder (30) verbunden ist, dass
eine Dorsalextensionsbewegung des Fußteils einen Strom von Hydraulikfluid (36) aus dem ersten Hydraulikzylinder (30), durch die erste Rückschlagarmatur (31) und in den zweiten Hydraulikzylinder (82) bewirkt und eine Plantarflexionsbewegung des Fußteils einen Strom von Hydraulikfluid aus dem zweiten Hydraulikzylinder (82), durch die zweite Rückschlagarmatur (82) und in den ersten Hydraulikzylinder (30) bewirkt.

12. Prothese nach einem der vorstehenden Ansprüche, **dadurch gekennzeichnet, dass** die die Freigabevorrichtung (16) eine Dämpfeinrichtung (59) umfasst, mittels der
- eine Dorsalextensionsbewegung des Verbindungselements (14) und/oder
- eine Plantarflexionsbewegung des Verbindungselements (14) dämpfbar ist.

13. Prothese nach einem der vorstehenden Ansprüche, **dadurch gekennzeichnet, dass**
(i) das Fußteil (18) relativ zum Unterschenkel-Anschlussteil (12)
- in eine Neutralstellung, in der eine die Prothese (10) tragende Person steht, und
- in eine Dorsalextensions-Stellung bringbar ist, und dass
(ii) die Prothese (10) eine mechanische Vorspannvorrichtung (62) aufweist, mittels der das Fußteil (18) in die Dorsalextensions-Stellung vorgespannt ist.

14. Verfahren zur passiven Steuerung einer Dorsalextension einer Prothese (10) nach einem der vorstehenden Ansprüche, **dadurch gekennzeichnet, dass** eine Freigabevorrichtung (16) passiv umgeschaltet wird, wenn eine zu einem Knöchel- Drehmoment (M) im Verbindungselement (14) korrelierende Größe einen voreingestellten Schwellenwert überschreitet.

15. Verfahren nach Anspruch 14, **dadurch gekennzeichnet, dass** die zu dem Knöchel- Drehmoment (M) im Verbindungselement (14) korrelierende Größe ein Knöchel- Drehmoment (M) und/oder eine Kraft und/oder eine Zeit und/oder eine Lage des Körperschwerpunkts ist.

## Claims

1. Prosthesis with
(a) a lower-leg connection part (12),
(b) a foot part (18),
(c) a connecting element (14) with a joint function that
connects the lower-leg connection part (12) with the foot part (18), and
(d) a release device (16) that
- can be brought into a locking position, in which the foot part (18) and the lower-leg connection part (12) are connected to one another in a rotationally fixed manner, at least with regards to a dorsal extension motion, and
- into a release position, in which the foot part (18) can be rotated relative to the lower-leg connection part (12),
**characterised by** the fact that
(e) the release device (16) is designed to passively switch from the locking position into the release position when a variable that correlates to an ankle torque (M) in the connecting element (14) lies above a pre-set threshold value.

2. Prosthesis according to claim 1, **characterised by** the fact that the release device (16) is designed to switch from the locking position into the release position when
the ankle torque (M) lies above a pre-set torque threshold value.

3. Prosthesis according claim 2, **characterised by** the fact that
the release device (16) is configured in such a way that
- an ankle torque (M) that lies above the torque threshold value moves the foot part (18) into a dorsally extended position, and that
- the foot part (18) remains at least predominantly in the dorsally extended position during a swing phase.

4. Prosthesis according to one of the above claims, **characterised by** the fact that the release device (16) comprises
(a) a hydraulic cylinder (30), which
- has a piston rod (32) and a cylinder housing (34) and
- whose end is connected to the foot part (18) and whose other end is connected to the lower-leg connection part (12), and
(b) an inhibiting device (29), which,
- when in a closing position, prevents a movement of the piston rod (32) that causes a dorsal extension motion of the foot part (18),
- when in an open position, enables the movement of the piston rod (32).

5. Prosthesis according to claim 4, **characterised by** the fact that the inhibiting device (29) has a check valve (31), in particular an excess pressure non-return valve (44) and/or a sequence valve (80) which,
- when in a closing position, prevents a movement of the piston rod (32) that causes a dorsal extension motion of the foot part (18),
- when in an open position, enables the dorsal extension motion of the piston rod (32), and
- has an opening pressure (pₒₚₑₙ) at which the check valve (31) moves out of the closing position into the open position.

6. Prosthesis according to one of the claims 4 to 5, **characterised by** the fact that the inhibiting device (29) comprises a mechanical inhibiting element (88).

7. Prosthesis according to claim 6, **characterised by** the fact that the release device (16) has an inhibiting structure (92) and a spring (94), wherein the inhibiting structure interacts with the inhibiting element (88) in such a way that a movement of the foot part beyond the zero position is only possible against the resistance of the spring (94).

8. Prosthesis according to claim 6 or 7, **characterised by** the fact that the release device (16) comprises
- a mechanical return element (106),
- a coupling ring (102) and
- a brake (108) that are connected to one another such that when the foot part (18) executes a dorsal extension motion, the non-return element (106) engages with the coupling ring (102) in such a way that the brake brakes the dorsal extension motion; and when the foot part (18) executes a plantar flexion motion, the brake (108) does not brake the plantar flexion motion as intensely, or does not brake it at all.

9. Prosthesis according to claim 4 to 6, **characterised by** the fact that the inhibiting device (29) has a second check valve (58), which,
- when in a closing position, prevents a plantar flexion motion of the foot part (18) and,
- when in an opening position, enables a plantar flexion motion of the foot part (18) and,
- has a plantar flexion opening pressure (p_{open,flex}) at which the second check valve (58) moves out of the closing position into the opening position.

10. Prosthesis according to claim 9, **characterised by** the fact that the hydraulic cylinder (30)
- is designed to be dual-acting and
- is connected to the inhibiting device (29) such that
a dorsal extension motion of the foot part (18) effects a flow of hydraulic fluid (36) through the first check valve (31) only, and
a plantar flexion motion of the foot part effects a flow of hydraulic fluid (36) through the second check valve (58) only.

11. Prosthesis according claim 9, **characterised by** the fact that
the release device (16) comprises a second hydraulic cylinder (82) which has
- a second piston rod (84) and a second cylinder housing (86) and
- whose one end is connected to the foot part (18) and whose other end is connected to the lower-leg connection part (12), and
- is connected to the inhibiting device (29) and the first hydraulic cylinder (30) such that
a dorsal extension motion of the foot part effects a flow of hydraulic fluid (36) out of the first hydraulic cylinder (30), through the first check valve (31) and into the second hydraulic cylinder (82), and
a plantar flexion motion of the foot part effects a flow of hydraulic fluid out of the second hydraulic cylinder (82), through the second check valve (82) and into the first hydraulic cylinder (30).

12. Prosthesis according to one of the above claims, **characterised by** the fact that the release device (16) comprises a damping device (59), by means of which
- a dorsal extension motion of the connecting element (14) and/or
- a plantar flexion motion of the connecting element (14)
can be dampened.

13. Prosthesis according to one of the above claims, **characterised by** the fact that
(i) the foot part (18) can be brought into
- a neutral position, in which a person wearing the prosthesis (10) is standing, and
- a dorsal extension position relative to the lower-leg connection part (12), and that
(ii) the prosthesis (10) comprises a mechanical pre-loading device (62), by means of which the foot part (18) is pre-loaded in the dorsal extension position.

14. Method for the passive control of a dorsal extension of a prosthesis (10) according to one of the above claims, **characterised by** the fact that a release device (16) is passively switched when a variable that correlates to an ankle torque (M) in the connecting element (14) lies above a pre-set threshold value.

15. Method according to claim 14, **characterised by** the fact that the variable that correlates to an ankle torque (M) in the connecting element (14) is an ankle torque (M) and/or a force and/or a time and/or a position of the body's centre of gravity.

## Revendications

1. Prothèse, comportant
(a) une partie (12) de raccordement à la jambe,
(b) une partie pied (18),
(c) un élément de liaison (14) à fonction d'articulation qui relie la partie (12) de raccordement à la jambe à la partie pied (18), et
(d) un dispositif de libération (16) qui peut être amené
- dans une position d'arrêt dans laquelle la partie pied (18) et la partie (12) de raccordement à la jambe sont reliées rigidement en rotation l'une à l'autre au moins par rapport à un mouvement d'extension dorsale,
- dans une position de libération dans laquelle la partie pied (18) est mobile en rotation par rapport à la partie (12) de raccordement à la jambe,
**caractérisée en ce que**
(e) le dispositif de libération (16) est réalisé pour la commutation passive depuis la position d'arrêt jusque dans la position de libération lorsqu'une grandeur en corrélation avec un couple de rotation de cheville (M) dans l'élément de liaison (14) est supérieure à une valeur seuil préréglée.

2. Prothèse selon la revendication 1, **caractérisée en ce que** le dispositif de libération (16) est réalisé pour la commutation depuis la position d'arrêt jusque dans la position de libération lorsque le couple de rotation de cheville (M) est supérieur à une valeur seuil de couple de rotation préréglée.

3. Prothèse selon la revendication 2, **caractérisée en ce que** le dispositif de libération (16) est réalisé de telle sorte que
- un couple de rotation de cheville (M) qui est supérieur à la valeur seuil de couple de rotation déplace la partie pied (18) jusque dans une position d'extension dorsale, et **en ce que**
- la partie pied (18) demeure au moins sensiblement dans la position d'extension dorsale pendant une phase d'élan.

4. Prothèse selon l'une des revendications précédentes, **caractérisée en ce que** le dispositif de libération (16) comprend
(a) un cylindre hydraulique (30) qui
- possède une tige de piston (32) et un boîtier cylindrique (34), et
- dont une extrémité est reliée à la partie pied (18) et dont l'autre extrémité est reliée à la partie (12) de raccordement à la jambe, et
(b) un dispositif inhibant (29) qui
- dans une position de fermeture, inhibe un mouvement de la tige de piston (32) qui mène à un mouvement d'extension dorsale de la partie pied (18),
- dans une position d'ouverture, permet le mouvement de la tige de piston (32).

5. Prothèse selon la revendication 4, **caractérisée en ce que** le dispositif inhibant (29) comprend une garniture anti-retour (31), en particulier un clapet anti-retour de surpression (44) et/ou une valve de séquence (80), qui
- dans une position de fermeture, inhibe un mouvement de la tige de piston (32) qui mène à un mouvement d'extension dorsale de la partie pied (18),
- dans une position d'ouverture, permet le mouvement d'extension dorsale de la tige de piston (32), et
- possède une pression d'ouverture (pₒₚₑₙ) à laquelle la garniture anti-retour (31) vient depuis la position de fermeture jusque dans la position d'ouverture.

6. Prothèse selon l'une des revendications 4 à 5, **caractérisée en ce que** le dispositif inhibant (29) comprend un élément inhibant mécanique (88).

7. Prothèse selon la revendication 6, **caractérisée en ce que** le dispositif de libération (16) comprend une structure inhibant (92) et un ressort (94), la structure inhibante coopérant avec l'élément inhibant (88) de telle sorte qu'un mouvement de la partie pied au-delà de la position zéro n'est possible qu'à l'encontre de la résistance d'un ressort (94).

8. Prothèse selon la revendication 6 ou 7, **caractérisée en ce que** le dispositif de libération (16) comprend
- un élément anti-retour mécanique (106),
- un anneau de couplage (102), et
- un frein (108),
qui sont reliés les uns aux autres de telle sorte que, lorsque la partie pied (18) exécute un mouvement d'extension dorsale, l'élément anti-retour (106) se couple avec l'anneau de couplage (102) de telle sorte que le frein freine le mouvement d'extension dorsale, et que, lorsque la partie pied (18) exécute un mouvement de flexion plantaire, le frein (108) ne freine pas ou freine moins fortement le mouvement de flexion plantaire que le mouvement d'extension dorsale.

9. Prothèse selon la revendication 4 à 6, **caractérisée en ce que** le dispositif inhibant (29) comprend une seconde garniture anti-retour (58), qui
- dans une position de fermeture, inhibe un mouvement de flexion plantaire de la partie pied (18), et
- dans une position d'ouverture, permet un mouvement de flexion plantaire de la partie pied (18), et
- possède une pression d'ouverture de flexion plantaire (p_{open,flex}) à laquelle la seconde garniture anti-retour (58) vient depuis la position de fermeture jusque dans la position d'ouverture.

10. Prothèse selon la revendication 9, **caractérisée en ce que** le cylindre hydraulique (30)
- est réalisé à effet double et
- est relié au dispositif inhibant (29) de telle sorte que
un mouvement d'extension dorsale de la partie pied (18) entraîne un flux de fluide hydraulique (36) uniquement à travers la première garniture anti-retour (31), et
un mouvement de flexion plantaire de la partie pied entraîne un flux de fluide hydraulique (36) uniquement à travers la seconde garniture anti-retour (58).

11. Prothèse selon la revendication 9, **caractérisée en ce que** le dispositif de libération (16) comprend un second cylindre hydraulique (82) qui
- possède une seconde tige de piston (84) et un second boîtier cylindrique (86) et
- dont une extrémité est reliée à la partie pied (18) et dont l'autre extrémité est reliée à la partie (12) de raccordement à la jambe, et
- est relié au dispositif inhibant (29) et au premier cylindre hydraulique (30) de telle sorte que
un mouvement d'extension dorsale de la partie pied entraîne un flux de fluide hydraulique (36) depuis le premier cylindre hydraulique (30) à travers la première garniture anti-retour (31) et jusque dans le second cylindre hydraulique (82) et
un mouvement d'extension plantaire de la partie pied entraîne un flux de fluide hydraulique depuis le second cylindre hydraulique (82) à travers la seconde garniture anti-retour (58) et jusque dans le premier cylindre hydraulique (30).

12. Prothèse selon l'une des revendications précédentes, **caractérisée en ce que** le dispositif de libération (16) comprend un moyen d'amortissement (59) permettant d'amortir
- un mouvement d'extension dorsale de l'élément de liaison (14) et/ou
- un mouvement de flexion plantaire de l'élément de liaison (14).

13. Prothèse selon l'une des revendications précédentes, **caractérisée en ce que**
(i) par rapport à la partie (12) de raccordement à la jambe, la partie pied (18) peut être amenée
- jusque dans une position neutre dans laquelle une personne portant la prothèse (10) est debout, et
- jusque dans une position d'extension dorsale,
et **en ce que**
(ii) la prothèse (10) comprend un dispositif de précontrainte mécanique (62) au moyen duquel la partie pied (18) est précontrainte dans la position d'extension dorsale.

14. Procédé pour la commande passive d'une extension dorsale d'une prothèse (10) selon l'une des revendications précédentes, **caractérisé en ce qu'**un dispositif de libération (16) est commuté de façon passive lorsqu'une grandeur en corrélation avec un couple de rotation de cheville (M) dans l'élément de liaison (14) dépasse une valeur seuil préréglée.

15. Procédé selon la revendication 14, **caractérisé en ce que** la grandeur en corrélation avec le couple de rotation de cheville (M) dans l'élément de liaison (14) est un couple de rotation de cheville (M) et/ou une force et/ou un temps et/ou une position du centre de gravité du corps.
